(19) Europäisches Patentamt
European Patent Office
Office européen des brevets

(11) EP 0 604 278 B1

(12) # FASCICULE DE BREVET EUROPEEN

(45) Date de publication et mention de la délivrance du brevet:
**21.02.2001 Bulletin 2001/08**

(51) Int Cl.$^7$: **C07D 209/08**, A61K 7/13

(21) Numéro de dépôt: **93403065.1**

(22) Date de dépôt: **17.12.1993**

(54) **Dérivés indoliniques et compositions tinctoriales pour fibres kératiniques**

Indoline Derivate und Färbemittel für Keratinfasern

Indoline derivatives and dye compositions for keratinic fibres

(84) Etats contractants désignés:
**AT BE CH DE DK ES FR GB GR IE IT LI NL PT SE**

(30) Priorité: **18.12.1992 FR 9215675**

(43) Date de publication de la demande:
**29.06.1994 Bulletin 1994/26**

(73) Titulaire: **L'OREAL**
**75008 Paris (FR)**

(72) Inventeurs:
- **Lagrange, Alain**
  **F-77700 Coupvray (FR)**
- **Vandenbosche, Jean-Jacques**
  **F-93270 Sevran (FR)**
- **Andrean, Hervé**
  **F-75014 Paris (FR)**

(74) Mandataire: **Casalonga, Axel**
**BUREAU D.A. CASALONGA - JOSSE**
**Morassistrasse 8**
**80469 München (DE)**

(56) Documents cités:

| | |
|---|---|
| **EP-A- 0 462 857** | **EP-A- 0 530 629** |
| **WO-A-92/17157** | **FR-A- 2 008 797** |
| **FR-A- 2 681 318** | **US-A- 4 428 881** |

- **CHEMICAL ABSTRACTS, vol. 86, 1977, Columbus, Ohio, US; abstract no. 139742n, MAZZA, M. ET AL.: 'N-acylindolines with phytotoxic activity Note II' page 535 ; & FARMACO, ED. SCI., vol.32, no.1, 1977 pages 54 - 66**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention est relative à des nouveaux composés indolines et à leur utilisation en teinture des fibres kératiniques, en particulier des fibres kératiniques humaines telles que les cheveux, aux compositions tinctoriales et aux procédés de teinture mettant en oeuvre ces indolines.

On a déjà proposé dans le passé de teindre les cheveux en utilisant comme coupleurs, certaines monohydroxyindolines ou monoaminoindolines, notamment dans le brevet français n° 2 008 797; le brevet US-A-4 013 404 décrit des mono- ou diamino indolines ou des monohydroxyindolines comme base d'oxydation ou comme coupleurs, utilisés en teinture d'oxydation des cheveux.

Le document Chemical Abstracts, vol. 86, 1977, Columbus, Ohio, US; abstract no. 139742n, Mazza, M. et Al et la demande US-A-4 428 881 décrivent certaines indolines utilisées comme herbicides.

La demanderesse a découvert de nouveaux composés du type indoline pouvant être utilisés pour la teinture des fibres kératiniques, en particulier des cheveux.

L'invention a donc pour objet l'utilisation pour la teinture des fibres kératiniques, en particulier des cheveux, de nouvelles indolinés et de leurs sels de formule (I) définie ci-après.

Un autre objet de l'invention est constitué par les compositions tinctoriales destinées à la teinture des fibres kératiniques et en particulier des cheveux humains, contenant au moins une indoline de formule (I) définie ci-après.

L'invention a également pour objet des procédés de teinture mettant en oeuvre ces composés.

D'autres objets de l'invention apparaîtront à la lecture de la description et des exemples qui suivent.

Les nouvelles indolines utilisées pour la teinture des fibres kératiniques et en particulier des fibres kératiniques humaines telles que les cheveux, conformément à l'invention, sont essentiellement caractérisées par le fait qu'elles répondent à la formule :

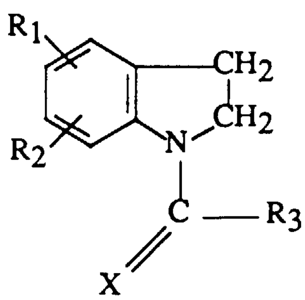

(I)

dans laquelle :

- $R_1$ et $R_2$, indépendamment l'un de l'autre, représentent un atome d'hydrogène, un groupement alkyle en $C_1$-$C_4$, monohydroxyalkyle en $C_1$-$C_4$, polyhydroxyalkyle en $C_2$-$C_4$, nitro, hydroxyle, alcoxy en $C_1$-$C_4$ ou NHR, R pouvant représenter un atome d'hydrogène, un groupement alkyle en $C_1$-$C_4$, monohydroxyalkyle en $C_1$-$C_4$ ou polyhydroxyalkyle en $C_2$-$C_4$;
- X représente un atome d'oxygène ou un atome de soufre;
- $R_3$ représente les groupements $-OR_4$ ou $NR_5R_6$, dans lesquels :
- $R_4$ représente un radical alkyle linéaire ou ramifié en $C_1$-$C_{10}$, alcényle linéaire ou ramifié en $C_2$-$C_{10}$, benzyle, alcoxyalkyle (les groupements alkyle et alcoxy ayant de 1 à 4 atomes de carbone) ou halogénoalkyle en $C_1$-$C_4$;
- $R_5$ et $R_6$, indépendamment l'un de l'autre, représentent un alkyle en $C_1$-$C_4$;

sous réserve que $R_1$ et $R_2$ ne peuvent pas désigner simultanément un atome d'hydrogène ou un groupement nitro et que lorsque $R_1$ désigne un radical alkyle, alcoxy, mono- ou polyhydroxyalkyle, $R_2$ représente un radical hydroxyle ou NHR, R ayant les significations indiquées ci-dessus; à l'exception des composés 6-nitro 1-N,N-diméthylcarbamoylindoline et 5-amino 1-N,N-diméthylcarbamoylindoline.

Parmi les composés répondant à la formule (I), on peut citer:

(1) la 5,6-dihydroxy 1-benzyloxycarbonylindoline,
(2) la 5,6-dihydroxy 1-éthyloxycarbonylindoline,
(3) la 5,6-dihydroxy 1-isopropyloxycarbonylindoline,
(4) la 5,6-dihydroxy 1-allyloxycarbonylindoline,

(5) la 5,6-dihydroxy 1-(2,2,2-trichloro)éthoxycarbonylindoline,
(6) la 5,6-dihydroxy 1-(2-éthyl)hexyloxycarbonylindoline,
(7) la 5,6-dihydroxy 1-méthoxyéthoxycarbonylindoline,
(8) la 5,6-dihydroxy 1-diméthylcarbamoylindoline,
(9) la 4-hydroxy 5-méthoxy 1-benzyloxycarbonylindoline,
(10) la 6-hydroxy 7-méthoxy 1-benzyloxycarbonylindoline,
(11) la 5,6-dihydroxy 1-éthylthiocarbonylindoline,
(12) la 5-méthoxy 6-hydroxy 1-benzyloxycarbonylindoline.

Parmi ces composés, les composés (1), (4), (5), (9), (10) et (12) sont préférés.

Les composés de formule (I) sont préparés selon un procédé de synthèse classique qui consiste à préparer, sous agitation, une suspension de bromhydrate d'indoline de formule :

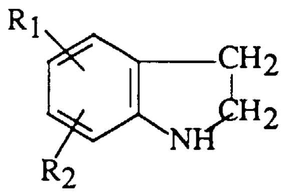 , HBr (II)

dans laquelle $R_1$ et $R_2$ ont les mêmes significations que dans la formule (I), et contenant du carbonate de calcium et du dioxane.

A température ambiante, on ajoute un chloroformiate ou chloroformamide correspondant au carbamate ou à l'amide que l'on souhaite obtenir.

On maintient l'agitation. Au bout de quelques minutes, la réaction étant exothermique, la température du mélange atteint 40/45°C. On maintient encore l'agitation pendant 5 à 20 minutes supplémentaires.

On verse ensuite la suspension sur de la glace. Il se forme alors un précipité que l'on acidifie avec de l'acide chlorhydrique. Le précipité est ensuite essoré puis lavé successivement à l'eau, à l'éthanol, à l'éther isopropylique et enfin à l'éther de pétrole. Une fois séché, le précipité est recristallisé dans l'acide acétique ou l'éthanol.

Les indolines de formule (I) définies ci-dessus, sont généralement mises en oeuvre à l'aide de compositions qui constituent un autre objet de l'invention.

Les compositions tinctoriales destinées à être utilisées pour la teinture des fibres kératiniques et en particulier des fibres kératiniques humaines telles que les cheveux, conformes à l'invention, sont caractérisées par le fait qu'elles contiennent dans un milieu approprié pour la teinture, au moins une indoline répondant à la formule (I) définie ci-dessus.

La quantité d'indoline de formule (I) utilisée dans la composition, est comprise généralement dans des proportions de 0,01 à 8% en poids par rapport au poids total de la composition et de préférence de 0,03 à 5% en poids.

Ces compositions peuvent se présenter sous des formes diverses, notamment sous forme de lotions plus ou moins épaissies, de crèmes, de mousses et de gels, éventuellement conditionnées sous forme d'aérosols.

Elles peuvent également constituer un élément d'un agent de teinture à plusieurs composant disposé dans un dispositif à plusieurs compartiments ou kit de teinture.

Le milieu approprié pour la teinture est de préférence un milieu aqueux qui doit être cosmétiquement acceptable lorsque les compositions sont destinées à être utilisées pour la teinture des cheveux humains vivants. Ce milieu aqueux peut être constitué par de l'eau ou un mélange eau/solvant(s).

Le pH des compositions est compris entre 4 et 12.

Les solvants sont choisis parmi les solvants organiques et préférentiellement parmi le diméthylisosorbide, les alcools en $C_2$-$C_4$, l'éthylèneglycol, les éthers monométhylique, monoéthylique et monobutylique de l'éthylèneglycol, l'acétate du monoéthyléther de l'éthylèneglycol, le propylèneglycol, les monométhyléthers du propylèneglycol et du dipropylèneglycol, le lactate de méthyle.

Un solvant particulièrement préféré est le mélange eau/diméthyl isosorbide.

Lorsque le milieu approprié pour la teinture est constitué par un mélange eau/solvant(s), les solvants sont utilisés dans des concentrations comprises entre 0,5 et 75% en poids par rapport au poids total de la composition.

Les compositions conformes à l'invention peuvent contenir des adjuvants habituellement utilisés pour la teinture des fibres kératiniques, et en particulier des adjuvants cosmétiquement acceptables lorsque ces compositions sont appliquées pour la teinture des cheveux humains vivants.

Ces compositions peuvent contenir notamment des amides gras dans des proportions préférentielles de 0,05

à 10% en poids, des agents tensio-actifs anioniques, cationiques, non-ioniques, amphotères ou leurs mélanges, présents de préférence dans des proportions comprises entre 0,1 et 50% en poids, des agents épaississants, des parfums, des agents séquestrants, des agents filmogènes, des agents de traitement, des agents dispersants, des agents de conditionnement, des agents conservateurs, des agents opacifiants, des agents de gonflement des fibres kératiniques.

Les agents épaississants sont choisis parmi l'alginate de sodium, la gomme arabique, la gomme de guar, les hétérobiopolysaccharides tels que la gomme de xanthane, les scléroglucanes, les dérivés de cellulose tels que la méthylcellulose, l'hydroxyéthylcellulose, l'hydroxypropylméthylcellulose, le sel de sodium de la carboxyméthylcellulose et les polymères d'acide acrylique réticulés de préférence.

On peut également utiliser les agents épaississants minéraux, tels que la bentonite.

Ces épaississants sont utilisés seuls ou en mélange et sont présents de préférence dans des proportions comprises entre 0,1 et 5% en poids par rapport au poids total de la composition et avantageusement entre 0,5 et 3% en poids.

Les agents alcalinisants utilisables dans les compositions peuvent être en particulier des amines, telles que les alcanolamines, des alkylamines, des hydroxydes ou carbonates alcalins ou d'ammonium.

Les agents d'acidification utilisables dans ces compositions peuvent être choisis parmi l'acide lactique, l'acide acétique, l'acide tartrique, l'acide phosphorique, l'acide chlorhydrique, l'acide citrique.

Il est bien entendu possible d'utiliser tout autre agent alcalinisant ou acidifiant acceptable, notamment dans le cas de la teinture des cheveux, en cosmétique.

Lorsque les compositions sont utilisées sous forme de mousse, elles peuvent être conditionnées sous pression et dans un dispositif aérosol en présence d'un agent propulseur et d'au moins un générateur de mousse.

Les agents générateurs de mousse peuvent être des polymères moussants anioniques, cationiques, non-ioniques, amphotères ou leurs mélanges, ou des agents tensio-actifs du type de ceux définis ci-dessus.

Le procédé de teinture des fibres kératiniques, en particulier des fibres kératiniques humaines, qui constitue un autre objet de l'invention, est essentiellement caractérisé par le fait qu'il consiste à appliquer sur ces fibres une composition (A) définie ci-dessus et contenant dans un milieu approprié pour la teinture, au moins une indoline de formule (I) définie ci-dessus, à maintenir la composition au contact des fibres pendant un temps suffisant pour développer la coloration, à l'aide d'un système oxydant, à rincer et éventuellement à laver les fibres ainsi teintes.

Selon l'invention, la couleur est révélée à l'aide d'un système oxydant chimique choisi parmi :

(i) des ions iodure et du peroxyde d'hydrogène, la composition (A) contenant l'indoline de formule (I) comprenant dans ce cas en plus, soit des ions iodure, soit du peroxyde d'hydrogène, et l'application de la composition (A) est précédée ou suivie par l'application d'une composition (B) qui contient dans un milieu approprié pour la teinture, soit :

  (a) du peroxyde d'hydrogène à un pH compris entre 2 et 12 et de préférence entre 2 et 7, lorsque la composition (A) contient des ions iodure, soit :
  (b) des ions iodure à un pH compris entre 3 et 11 lorsque la composition (A) contient du peroxyde d'hydrogène;

(ii) des nitrites, l'application de la composition (A) contenant l'indoline de formule (I) étant suivie par l'application d'une composition aqueuse (B) présentant un pH acide, la composition (A) ou la composition (B) contenant au moins un nitrite;
(iii) des oxydants choisis parmi le peroxyde d'hydrogène, l'acide periodique et ses sels hydrosolubles, le métaperiodate de sodium, l'hypochlorite de sodium, la chloramine T, la chloramine B, le ferricyanure de potassium, l'oxyde d'argent, le réactif de Fenton, l'oxyde de plomb (IV), le sulfate de cesium, le persulfate d'ammonium, les chlorites alcalins; ces oxydants étant présents dans la composition (A) contenant l'indoline de formule (I) ou appliqués simultanément ou séquentiellement au moyen d'une composition (B) les contenant dans un milieu approprié pour la teinture;
(iv) des anions d'un métal choisis parmi les permanganates ou bichromates, ces oxydants étant appliqués au moyen d'une composition (B) aqueuse, à un pH de 2 à 10, avant l'application de la composition (A);
(v) des sels métalliques des groupes 3 à 8 du tableau périodique, ces sels métalliques étant appliqués au moyen d'une composition (B) les contenant dans un milieu approprié pour la teinture, la composition (B) étant appliquée préalablement ou postérieurement à l'application de la composition (A);
(vi) des sels de terres rares, ces sels de terres rares étant appliqués au moyen d'une composition (B) les contenant dans un milieu approprié pour la teinture, la composition (B) étant appliquée préalablement ou postérieurement à l'application de la composition (A) contenant l'indoline de formule (I);
(vii) un dérivé quinonique choisi parmi les ortho- ou parabenzoquinones, les ortho- ou parabenzoquinones monoïmines ou diimines, les 1,2- ou 1,4-naphtoquinones, les ortho- ou parabenzoquinones sulfonimides, les $\alpha$, $\omega$-alkylène-bis-1,4-benzoquinones;

ou les 1, 2 ou 1,4-naphtoquinones monoïmines ou diimines, l'indoline de formule (I) et les dérivés quinoniques étant choisis de façon à ce que la différence de potentiel d'oxydoréduction ΔE entre le potentiel d'oxydoréduction Ei de l'indoline de formule (I) déterminé à pH 7 en milieu phosphate sur électrode de carbone vitreux par voltamétrie et le potentiel d'oxydoréduction Eq du dérivé quinonique déterminé à pH 7 en milieu phosphate par polarographie sur électrode de mercure par rapport à l'électrode au calomel saturé, soit telle que :

$$\Delta E = Ei - Eq \leq 320 \text{ millivolts.}$$

Selon une forme préférée de l'invention, l'application des compositions (A) et (B) est séparée par une étape de rinçage à l'eau.

Selon une première variante du procédé de teinture mettant en oeuvre des systèmes oxydants, on applique sur les matières kératiniques une composition (A) contenant dans un milieu approprié pour la teinture, au moins un composé de formule (I) en association avec des ions iodure, l'application de la composition (A) étant précédée ou suivie par l'application d'une composition (B) qui contient dans un milieu approprié pour la teinture, du peroxyde d'hydrogène.

Ce procédé peut également être mis en oeuvre en appliquant sur les fibres kératiniques, au moins une composition (A) contenant dans un milieu approprié pour la teinture, le composé de formule (I) en association avec du peroxyde d'hydrogène, ayant un pH compris entre 2 et 7, et de préférence entre 3, 5 et 7, l'application de la composition (A) étant précédée ou suivie par l'application d'une composition (B) qui contient dans un milieu approprié pour la teinture, des ions iodure.

L'ion iodure dans cette variante du procédé, est choisi de préférence parmi les iodures de métaux alcalins, alcalino-terreux ou d'ammonium. L'iodure est plus particulièrement constitué par l'iodure de potassium.

Les ions iodure sont présents dans les compositions (A) ou (B) dans des proportions comprises généralement entre 0,007 et 4% en poids exprimées en ions $I^-$ et de préférence entre 0,08 et 1,5% en poids par rapport au poids total de la composition (A) ou (B).

Selon une seconde variante, ce procédé peut être mis en oeuvre en utilisant comme agent oxydant pour révéler la coloration, un nitrite. Les nitrites plus particulièrement utilisables conformément à l'invention, sont :

- des nitrites de métaux alcalins, alcalino-terreux ou d'ammonium ou de tout autre cation cosmétiquement acceptable lorsqu'il est utilisé pour la teinture des cheveux humains vivants;
- des dérivés organiques des nitrites tels que par exemple le nitrite d'amyle;
- des vecteurs de nitrites, c'est-à-dire des composés qui, par transformation, forment des nitrites du type défini ci-dessus.

Les nitrites particulièrement préférés sont les nitrites de sodium, de potassium ou d'ammonium.

Cette variante du procédé est mise en oeuvre en appliquant sur les matières kératiniques, la composition (A) à base du composé de formule (I) définie ci-dessus, puis celle d'une composition aqueuse acide (B), la composition (A) ou (B) contenant au moins un nitrite.

Les nitrites sont généralement utilisés dans des proportions comprises entre 0,02 et 1 mole/litre.

Selon une troisième variante de ce procédé, les oxydants sont choisis parmi le peroxyde d'hydrogène, la chloramine T, la chloramine B, l'acide periodique et ses sels hydrosolubles, le metapériodate de sodium, l'hypochlorite de sodium, le ferricyanure de potassium, l'oxyde d'argent, le réactif de Fenton, l'oxyde de plomb (IV), le sulfate de cesium, le persulfate d'ammonium. Ces agents sont de préférence appliqués sur les fibres au moyen d'une composition (B) et après l'application de la composition (A).

Ces agents oxydants sont présents dans des proportions suffisantes pour développer une coloration et de préférence dans des proportions comprises entre 0,004 mole et 0,7 mole, en particulier entre 0,01 mole et 0,04 mole pour 100 g de composition.

Selon cette troisième variante, le métaperiodate de sodium est particulièrement préféré.

Selon une quatrième variante de ce procédé, on applique dans un premier temps sur les fibres kératiniques, une composition contenant dans un milieu approprié pour la teinture, à un pH compris entre 2 et 10, un anion d'un métal ayant une bonne affinité pour la kératine ayant un potentiel d'oxydoréduction supérieur à celui des composés de formule (I). Cet anion est choisi de préférence parmi les permanganates ou les bichromates et plus particulièrement le permanganate de potassium et le bichromate de sodium.

Ces anions métalliques sont généralement utilisés à des molalités en anions supérieures à $10^{-3}$ mole/1000 g jusqu'à de préférence de 1 mole/1000 g.

Dans un second temps, on applique une composition contenant dans un milieu approprié pour la teinture, à un pH compris entre 4 et 10, un composé répondant à la formule (I) définie ci-dessus.

Les compositions contenant les anions ne doivent pas contenir d'agents organiques ayant un effet réducteur sur les anions.

Selon une cinquième variante de l'invention, on met en oeuvre des catalyseurs d'oxydation choisis parmi les sels métalliques tels que les sels de manganèse, de cobalt, de fer, de cuivre et d'argent.

A titre d'exemple, on peut utiliser le sulfate de manganèse, le lactate de manganèse, le chlorure de cobalt, le chlorure ferrique, le chlorure cuivrique, le nitrate d'argent ammoniacal, le sulfate de cuivre.

Les sels préférés sont les sels de cuivre. Ces sels sont utilisés dans des proportions de 0,01 à 2% exprimées en ions métalliques par rapport au poids total de la composition mise en oeuvre et contenant ces sels.

Selon cette variante, on met les fibres kératiniques, en particulier les cheveux, en contact avec une composition (B) contenant dans un milieu approprié pour la teinture, le sel métallique, avant ou après l'application de la composition (A) contenant le composé de formule (I) et on rince de préférence entre les deux étapes.

La forme de réalisation préférée consiste à appliquer un sel cuivrique dans un premier temps, et la composition (A) contenant l'indoline de formule (I), dans un deuxième temps.

Selon cette variante, le sulfate de cuivre est particulièrement préféré.

On peut faire suivre cette teinture après rinçage, de l'application d'une solution de peroxyde d'hydrogène.

Selon une sixième variante, on utilise des sels de terres rares. Les sels de terres rares utilisables conformément à l'invention, sont choisis parmi les sels de Lanthanides, et notamment les sels de Cérium $Ce^{3+}$, $Ce^{4+}$, de Lanthane $La^{3+}$, d'Europium $Eu^{2+}$, $Eu^{3+}$, de Gadolinium $Gd^{3+}$, d'Ytterbium $Yb^{2+}$, $Yb^{3+}$, de Dysprosium $Dy^{3+}$. Les sels préférés sont en particulier les sulfates, chlorures ou nitrates.

Ces sels de terres rares sont présents dans des proportions comprises entre 0,1 et 8% en poids par rapport au poids total de la composition.

On utilise de préférence les sels de Cérium $Ce^{3+}$, $Ce^{4+}$ sous la forme de sulfates et de chlorures.

Selon une septième variante, la composition contenant le dérivé quinonique est appliquée avant ou après la composition (A) contenant le composé de formule (I).

A titre d'exemple de dérivé quinonique, on peut citer la 1,4-benzoquinone et la 2-hydroxyéthylthio 1,4-benzoquinone.

La concentration en dérivés quinoniques est de préférence comprise entre 0,005 et 1 mole/litre dans la composition (B). Le pH de la composition (B) est compris entre 2 et 10 et de préférence inférieur à 7.

Lorsqu'on met en oeuvre des compositions à base de peroxyde d'hydrogène dans les différents procédés décrits ci-dessus, la teneur en peroxyde d'hydrogène est généralement comprise entre 1 et 40 volumes et de préférence entre 2 et 10 volumes et plus particulièrement entre 3 et 10 volumes.

L'invention a également pour objet un agent de coloration des fibres kératiniques et en particulier des fibres kératiniques humaines, à plusieurs composants, destiné notamment à être utilisé dans la mise en oeuvre du procédé de teinture défini ci-dessus et utilisant un système oxydant. Dans ce cas, l'agent de teinture comprend au moins deux composants dont un premier est constitué par la composition (A) définie ci-dessus et contenant l'indoline de formule (I) et l'autre composant est constitué par l'une des compositions (B) également définie ci-dessus.

Les composants (A) et (B) respectifs sont choisis selon les différentes variantes du procédé exposées ci-dessus.

L'invention a également pour objet un dispositif à plusieurs compartiments ou encore "kit de teinture" ou "nécessaire de teinture", comportant tous les composants destinés à être appliqués dans une même teinture sur les fibres kératiniques en applications uniques ou successives avec ou sans prémélange comme mentionné ci-dessus.

De tels dispositifs sont connus en eux-mêmes et peuvent comporter un premier compartiment contenant la composition (A) contenant l'indoline de formule (I) dans un milieu approprié pour la teinture, et dans un second compartiment une composition (B) du type défini ci-dessus et contenant l'agent oxydant.

Les dispositifs à plusieurs compartiments utilisables conformément à l'invention, peuvent être équipés des moyens de mélanges au moment de l'emploi et leur contenu peut être conditionné sous atmosphère inerte.

Lorsque le milieu contenant l'indoline de formule (I) est anhydre, on peut prévoir un troisième compartiment contenant un milieu aqueux approprié pour la teinture et destiné à être mélangé tout juste avant l'emploi avec la composition du premier compartiment.

L'indoline de formule (I), les compositions et le procédé conformes à l'invention, peuvent être mis en oeuvre pour teindre des cheveux naturels ou déjà teints, permanentés ou non, défrisés ou non, ou des cheveux fortement ou légèrement décolorés et éventuellement permanentés.

Il est également possible de les utiliser pour la teinture de la fourrure ou de la laine.

Les exemples sont destinés à illustrer l'invention sans pour autant présenter un caractère limitatif.

**EXEMPLES DE PREPARATION**

EXEMPLE 1

Préparation de la 5,6-dihydroxy 1-benzyloxycarbonylindoline.

On prépare une suspension de bromhydrate de 5,6-dihydroxyindoline 69,6 g (0,3 M) et de carbonate de calcium (0,6 M, soit 60 g) dans 345 ml de dioxane. On ajoute 90 ml d'eau et on agite pendant 3 minutes. On ajoute ensuite, toujours sous agitation, en une seule fois, et à température ambiante, 56,1 g (0,33 M) de chloroformiate de benzyle. On maintient l'agitation pendant environ 20 minutes. La réaction est exothermique (40/45°C). La suspension est ensuite versée sur de la glace. Il se forme un précipité blanc qui est acidifié par de l'acide chlorhydrique puis essoré et lavé successivement à l'eau, l'éthanol, l'éther isopropylique, l'éther de pétrole. Le précipité est séché et recristallisé de l'éthanol.

On obtient avec un rendement de 79% des cristaux blancs du produit attendu dont l'analyse élémentaire est la suivante :

| | C | H | N | O |
|---|---|---|---|---|
| Calculé | 67,36 | 5,30 | 4,91 | 22,43 |
| Trouvé | 67,33 | 5,25 | 5,02 | 22,55 |

EXEMPLE 2

Préparation de la 5,6-dihydroxy 1-éthyloxycarbonylindoline.

On prépare une suspension de bromhydrate de 5,6-dihydroxyindoline 34,8 g (0,15 M) et de carbonate de calcium 30 g (0,3 M) dans 120 ml de dioxane. On ajoute 10 ml d'eau et on agite pendant 3 minutes. On procède ensuite comme à l'exemple 1, sauf que l'on ajoute 15,9 g (0,165 M) de chloroformiate d'éthyle.

On obtient avec un rendement de 81% des cristaux blancs du produit attendu dont l'analyse élémentaire est la suivante :

| | C | H | N | O |
|---|---|---|---|---|
| Calculé | 59,19 | 5,87 | 6,27 | 28,67 |
| Trouvé | 59,06 | 5,88 | 6,21 | 28,95 |

EXEMPLE 3

Préparation de la 5,6-dihydroxy 1-isopropyloxycarbonylindoline.

On procède comme à l'exemple 2, sauf que l'on utilise 21,3 g (0,165 M) de chloroformiate d'isopropyle.

On obtient avec un rendement de 67% des cristaux blancs du produit attendu dont l'analyse élémentaire est la suivante :

| | C | H | N | O |
|---|---|---|---|---|
| Calculé | 62,14 | 6,82 | 5,57 | 25,47 |
| Trouvé | 62,08 | 6,85 | 5,53 | 25,58 |

EXEMPLE 4

Préparation de la 5,6-dihydroxy 1-allyloxycarbonylindoline.

On procède comme à l'exemple 2, sauf que l'on utilise 17,4 g (0,165 M) de chloroformiate d'allyle.

On obtient avec un rendement de 88% des cristaux blancs du produit attendu dont l'analyse élémentaire est la suivante :

| | C | H | N | O |
|---|---|---|---|---|
| Calculé<br>Trouvé | 61,27<br>61,11 | 5,57<br>5,61 | 5,95<br>5,86 | 27,21<br>27,27 |

EXEMPLE 5

Préparation de la 5,6-dihydroxy 1-(2,2,2-trichloro)éthoxycarbonylindoline.

On procède comme à l'exemple 2, sauf que l'on utilise 35 g (0,165 M) de chloroformiate de 2,2,2-trichloroéthyle.

On obtient avec un rendement de 75% des cristaux blancs du produit attendu dont l'analyse élémentaire est la suivante :

| | C | H | N | O | Cl |
|---|---|---|---|---|---|
| Calculé<br>Trouvé | 40,46<br>40,52 | 3,09<br>3,07 | 4,29<br>4,05 | 19,60<br>19,61 | 32,57<br>32,44 |

EXEMPLE 6

Préparation de la 5,6-dihydroxy 1-(2-éthyl)hexyloxycarbonylindoline.

On procède comme indiqué à l'exemple 2, sauf que l'on utilise 31,8 g (0,165 M) de chloroformiate de 2-éthylhexyle.

On obtient avec un rendement de 40% des cristaux blancs du produit attendu dont l'analyse élémentaire est la suivante :

| | C | H | N | O |
|---|---|---|---|---|
| Calculé<br>Trouvé | 66,43<br>66,51 | 8,18<br>8,18 | 4,56<br>4,52 | 20,82<br>20,71 |

EXEMPLE 7

Préparation de la 5,6-dihydroxy 1-méthoxyéthoxycarbonylindoline.

On procède comme indiqué à l'exemple 2, sauf que l'on utilise 24,9 g (0,18 M) de chloroformiate de méthoxyéthyle.

On obtient avec un rendement de 80% des cristaux blancs du produit attendu dont l'analyse élémentaire est la suivante :

| | C | H | N | O |
|---|---|---|---|---|
| Calculé<br>Trouvé | 56,91<br>56,86 | 5,97<br>6,09 | 5,53<br>5,45 | 31,59<br>31,53 |

EXEMPLE 8

Préparation de la 5,6-dihydroxy 1-diméthylcarbamoylindoline.

On procède comme indiqué à l'exemple 1, sauf que l'on utilise :

- 46,4 g (0,2 M) de 5,6-dihydroxyindoline,
- 40 g (0,4 M) de carbonate de calcium,
- 300 ml de dioxane,
- 60 ml d'eau,

- 38,7 g (0,36 M) de chloroformamide de diméthyle.

On obtient avec un rendement de 16% des cristaux blancs du produit attendu dont l'analyse élémentaire est la suivante :

| | C | H | N | O |
|---|---|---|---|---|
| Calculé | 59,46 | 6,35 | 12,60 | 21,60 |
| Trouvé | 58,88 | 6,52 | 12,32 | 22,32 |

EXEMPLE 9

Préparation de la 4-hydroxy 5-méthoxy 1-benzyloxycarbonylindoline.

On procède comme indiqué à l'exemple 1, sauf que l'on utilise :

- 30,2 g (0,15 M) de 4-hydroxy 5-méthoxyindoline,
- 15,0 g (0,15 M) de carbonate de calcium,
- 130 ml de dioxane,
- 12 ml d'eau,
- 12,5 ml (0,15 M) d'ammoniaque (que l'on ajoute en même temps que l'eau),
- 24,0 g (0,17 M) de chloroformiate de benzyle.

On obtient avec un rendement de 85% des cristaux blancs du produit attendu cristallisé avec 1/4 d'$H_2O$, dont l'analyse élémentaire est la suivante :

| | C | H | N | O |
|---|---|---|---|---|
| Calculé | 67,22 | 5,76 | 4,61 | 22,40 |
| Trouvé | 67,26 | 6,03 | 4,38 | 22,55 |

EXEMPLE 10

Préparation de la 6-hydroxy 7-méthoxy 1-benzyloxycarbonylindoline.

On procède comme indiqué à l'exemple 9, sauf que l'on utilise :

- 16,1 g (0,08 M) de 6-hydroxy 7-méthoxyindoline,
- 8 g (0,08 M) de carbonate de calcium,
- 64 ml de dioxane,
- 6 ml d'eau,
- 6 ml d'ammoniaque (0,08 M),
- 15 g (0,088 M) de chloroformiate de benzyle.

On obtient avec un rendement de 88% des cristaux blancs du produit attendu dont l'analyse élémentaire est la suivante :

| | C | H | N | O |
|---|---|---|---|---|
| Calculé | 68,22 | 5,72 | 4,68 | 21,38 |
| Trouvé | 68,12 | 5,64 | 4,65 | 21,29 |

EXEMPLE 11

Préparation de la 5,6-dihydroxy 1-éthylthiocarbonylindoline.

On procède comme indiqué à l'exemple 2, sauf que l'on ajoute 18,3 g (0,165 M) de chloroformiate de thioéthyle.

On obtient avec un rendement de 77% des cristaux blancs du produit attendu dont l'analyse élémentaire est la suivante :

| | C | H | N | O | S |
|---|---|---|---|---|---|
| Calculé | 55,21 | 5,48 | 5,85 | 20,06 | 13,40 |
| Trouvé | 55,25 | 5,46 | 5,79 | 20,29 | 13,18 |

**EXEMPLES DE TEINTURE**

EXEMPLE 1

On prépare les compositions suivantes :

**COMPOSITION (A)**

- Sulfate de cuivre pentahydrate 1 g
- Lauryléthersulfate de sodium, vendu sous la dénomination "EMPICOL ESB/3 FL" par la Société MARCHON 4,2 g MA
- Hydroxyéthylcellulose vendue sous la dénomination "CELLOSIZE WP 34" par la Société UNION CARBIDE 2,4 g MA
- Monoéthanolamine qs pH = 9,5
- Eau qsp 100g

**COMPOSITION (B)**

- 5,6-dihydroxy 1-benzyloxycarbonyl indoline 0,5 g
- Diméthylisosorbide 15 g
- Eau 10 g
- Triéthanolamine qs pH = 7,8

On applique la composition (A) sur des cheveux gris naturels à 90% de blancs pendant 10 minutes, puis on rince les cheveux. On applique ensuite la solution (B) contenant l'indoline pendant 10 minutes. Les cheveux sont ensuite rincés et séchés. Les cheveux sont colorés dans une nuance blond très clair cendré doré.

EXEMPLE 2

On prépare les compositions suivantes :

**COMPOSITION (A)**

- 5,6-dihydroxy 1-benzyloxycarbonyl indoline 0,5 g
- Diméthylisosorbide 15 g
- Eau 10 g
- Triéthanolamine qs pH = 7,8

**COMPOSITION (B)**

- Métapériodate de sodium 5 g
- Acide chlorhydrique qs pH = 3

- Eau qsp 100 g

On applique la composition (A) sur des cheveux gris permanentés à 90% de blancs pendant 15 minutes, puis on rince les cheveux. On applique ensuite la composition (B) pendant 15 minutes. Les cheveux sont ensuite rincés et séchés.
Les cheveux sont colorés dans une nuance blond clair doré.

EXEMPLES 3 à 10

On prépare les compositions suivantes :

**COMPOSITION (A)**

- Sulfate de cuivre, pentahydrate 1 g
- Lauryléthersulfate de sodium, vendu sous la dénomination "EMPICOL ESB/3 FL" par la Société MARCHON 4,2 g MA
- Hydroxyéthylcellulose vendue sous la dénomination "CELLOSIZE WP 34" par la Société UNION CARBIDE 2,4 g MA
- Monoéthanolamine qs pH = 9,5
- Eau qsp 100 g

**COMPOSITION (B)**

- Indoline de formule (I) 0,5 g
- Diméthylisosorbide 15 g
- Eau 10 g
- Triéthanolamine qs pH = 7,8

On applique la composition (A) pendant 10 minutes sur des cheveux gris à 90% de blancs naturels ou permanentés, puis on rince les cheveux.
On applique ensuite la composition (B) contenant l'indoline pendant 10 minutes. Les cheveux sont ensuite rincés et séchés.
Les couleurs obtenues sont présentées dans le tableau ci-après :

TABLEAU

| EXEMPLES | INDOLINE | COULEUR SUR CHEVEUX GRIS NATURELS A 90% BLANCS | COULEUR SUR CHEVEUX GRIS PERMANENTES A 90% BLANCS |
|---|---|---|---|
| 3 | 5,6-dihydroxy 1-éthyloxycarbonylindoline | Cendré doré mat | |
| 4 | 5,6-dihydroxy 1-isopropyloxycarbonylindoline | | Cendré doré mat |
| 5 | 5,6-dihydroxy 1-allyloxycarbonylindoline | Cendré doré mat | |
| 6 | 5,6-dihydroxy 1-(2-éthyl) hexyloxycarbonylindoline | | Cendré doré mat |
| 7 | 5,6-dihydroxy 1-(2,2,2-trichloro) éthoxycarbonylindoline | Cendré doré mat | |
| 8 | 4-hydroxy 5-méthoxy 1-benzyloxycarbonylindoline | | Cendré doré mat |

TABLEAU (suite)

| EXEMPLES | INDOLINE | COULEUR SUR CHEVEUX GRIS NATURELS A 90% BLANCS | COULEUR SUR CHEVEUX GRIS PERMANENTES A 90% BLANCS |
|---|---|---|---|
| 9 | 6-hydroxy 7-méthoxy 1-benzyloxycarbonylindoline | Cendré doré mat | |
| 10 | 5,6-dihydroxy 1-méthoxyéthoxycarbonylindoline | | Cendré doré mat |

EXEMPLES 11 à 18

On prépare les compositions suivantes :

**COMPOSITION (A)**

- Indoline de formule (I) 0,5 g
- Diméthylisosorbide 15 g
- Eau 10 g
- Triéthanolamine qs pH = 7,8

**COMPOSITION (B)**

- Métaperiodate de sodium 5 g
- HCl qs pH = 3
- Eau qsp 100 g

On applique la composition (A) pendant 15 minutes sur des cheveux gris à 90% de blancs naturels ou permanentés, puis on rince les cheveux. On applique ensuite la composition (B) pendant 15 minutes. Les cheveux sont ensuite rincés et séchés.
Les couleurs obtenues sont présentées dans le tableau ci-après :

TABLEAU

| EXEMPLES | INDOLINE | COULEUR SUR CHEVEUX GRIS NATURELS A 90% BLANCS | COULEUR SUR CHEVEUX GRIS PERMANENTES A 90% BLANCS |
|---|---|---|---|
| 11 | 5,6-dihydroxy 1-éthyloxycarbonylindoline | Beige doré | |
| 12 | 5,6-dihydroxy 1-isopropyloxycarbonylindoline | | Beige doré |
| 13 | 5,6-dihydroxy 1-allyloxycarbonylindoline | Beige doré | |
| 14 | 5,6-dihydroxy 1-(2-éthyl) hexyloxycarbonylindoline | | Beige doré légèrement nacré |
| 15 | 5,6-dihydroxy 1-(2,2,2-trichloro) éthoxycarbonylindoline | Beige doré | |
| 16 | 4-hydroxy 5-méthoxy 1-benzyloxycarbonylindoline | | Beige doré |

TABLEAU (suite)

| EXEMPLES | INDOLINE | COULEUR SUR CHEVEUX GRIS NATURELS A 90% BLANCS | COULEUR SUR CHEVEUX GRIS PERMANENTES A 90% BLANCS |
|---|---|---|---|
| 17 | 6-hydroxy 7-méthoxy 1-benzyloxycarbonylindoline | Beige doré | |
| 18 | 5,6-dihydroxy 1-méthoxyéthoxycarbonylindoline | | Beige doré |

**Revendications**

**1.** Composé indoline de formule :

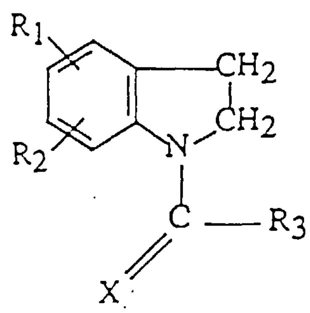

(I)

dans laquelle :

$R_1$ et $R_2$, indépendamment l'un de l'autre, représentent un atome d'hydrogène, un groupement alkyle en $C_1$-$C_4$, monohydroxyalkyle en $C_1$-$C_4$, polyhydroxyalkyle en $C_2$-$C_4$, nitro, hydroxyle, alcoxy en $C_1$-$C_4$ ou NHR, R pouvant représenter un atome d'hydrogène, un groupement alkyle en $C_1$-$C_4$, monohydroxyalkyle en $C_1$-$C_4$ ou polyhydroxyalkyle en $C_2$-$C_4$;
X représente un atome d'oxygène ou un atome de soufre;
$R_3$ représente les groupements $-OR_4$ ou $NR_5R_6$, dans lesquels :
$R_4$ représente un radical alkyle linéaire ou ramifié en $C_1$-$C_{10}$, alcényle linéaire ou ramifié en $C_2$-$C_{10}$, benzyle, alcoxyalkyle (les groupements alkyle et alcoxy ayant de 1 à 4 atomes de carbone) ou halogénoalkyle en $C_1$-$C_4$;
$R_5$ et $R_6$, indépendamment l'un de l'autre, représentent un alkyle en $C_1$-$C_4$;

sous réserve que $R_1$ et $R_2$ ne peuvent pas désigner simultanément un atome d'hydrogène ou un groupement nitro et que lorsque $R_1$ désigne un radical alkyle, alcoxy, mono- ou polyhydroxyalkyle, $R_2$ représente un radical hydroxyle ou NHR, R ayant les significations indiquées ci-dessus; à l'exception des composés 6-nitro 1-N,N-diméthylcarbamoylindollne;
5-nitro 1-N,N-diméthyl carbamoylindoline et 5-amino 1-N,N-diméthylcarbamoylindoline.

**2.** Composés selon la revendication 1, caractérisés par le fait qu'ils sont choisis parmi :

- la 5,6-dihydroxy 1-benzyloxycarbonylindoline,
- la 5,6-dihydroxy 1-éthyloxycarbonylindoline,
- la 5,6-dihydroxy 1-isopropyloxycarbonylindoline,
- la 5,6-dihydroxy 1-allyloxycarbonylindoline,
- la 5,6-dihydroxy 1-(2,2,2-trichloro)éthoxycarbonylindoline,
- la 5,6-dihydroxy 1-(2-éthyl)hexyloxycarbonylindoline,
- la 5,6-dihydroxy 1-méthoxyéthoxycarbonylindoline,
- la 5,6-dihydroxy 1-diméthylcarbamoylindoline,
- la 4-hydroxy 5-méthoxy 1-benzyloxycarbonylindoline,
- la 6-hydroxy 7-méthoxy 1-benzyloxycarbonylindoline,

- la 5,6-dihydroxy 1-éthylthiocarbonylindoline,
- la 5-méthoxy 6-hydroxy 1-benzyloxycarbonylindoline.

**3.** Composition tinctoriale destinée à être utilisée pour la teinture des fibres kératiniques, en particulier des fibres kératiniques humaines, caractérisée par le fait qu'elle contient dans un milieu approprié pour la teinture, au moins une indoline de formule :

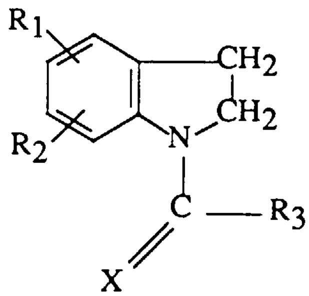

(I)

dans laquelle :

$R_1$ et $R_2$, indépendamment l'un de l'autre, représentent un atome d'hydrogène, un groupement alkyle en $C_1$-$C_4$, monohydroxyalkyle en $C_1$-$C_4$, -polyhydroxyalkyle en $C_2$-$C_4$, nitro, hydroxyle, alcoxy en $C_1$-$C_4$ ou NHR, R pouvant représenter un atome d'hydrogène, un groupement alkyle en $C_1$-$C_4$, monohydroxyalkyle en $C_1$-$C_4$ ou polyhydroxyalkyle en $C_2$-$C_4$;
X représente un atome d'oxygène ou un atome de soufre;
$R_3$ représente les groupements $-OR_4$ ou $NR_5R_6$, dans lesquels :
$R_4$ représente un radical alkyle linéaire ou ramifié en $C_1$-$C_{10}$, alcényle linéaire ou ramifié en $C_2$-$C_{10}$, benzyle, alcoxyalkyle (les groupements alkyle et alcoxy ayant de 1 à 4 atomes de carbone) ou halogénoalkyle en $C_1$-$C_4$;
$R_5$ et $R_6$, indépendamment l'un de l'autre, représentent un alkyle en $C_1$-$C_4$;

sous réserve que $R_1$ et $R_2$ ne peuvent pas désigner simultanément un atome d'hydrogène ou un groupement nitro et que lorsque $R_1$ désigne un radical alkyle, alcoxy, mono- ou polyhydroxyalkyle, $R_2$ représente un radical hydroxyle ou NHR, R ayant les significations indiquées ci-dessus.

**4.** Composition selon la revendication 3, caractérisée par le fait que l'indoline est présente dans la composition dans des proportions comprises entre 0,01 et 8% en poids par rapport au poids total de la composition et de préférence entre 0,03 et 5% en poids.

**5.** Composition selon la revendication 3 ou 4, caractérisée par le fait que le milieu approprié pour la teinture est un milieu aqueux constitué par de l'eau ou un mélange eau/solvant(s).

**6.** Composition selon l'une quelconque des revendications 3 à 5, caractérisée par le fait que la composition contient en outre au moins un adjuvant choisi parmi les amides gras, les agents tensio-actifs anioniques, cationiques, non-ioniques, amphotères ou leurs mélanges, les agents épaississants, les parfums, les agents séquestrants, les agents filmogènes, les agents de traitement, les agents dispersants, les agents de conditionnement, les agents conservateurs, les agents opacifiants, les agents de gonflement des fibres kératiniques ou leurs mélanges.

**7.** Composition selon l'une quelconque des revendications 3 à 6, caractérisée par le fait que le pH de la composition est compris entre 4 et 12.

**8.** Procédé de teinture des fibres kératiniques, en particulier des fibres kératiniques humaines, caractérisé par le fait que l'on applique sur ces fibres au moins une composition telle que définie dans l'une quelconque des revendications 3 à 7, que l'on maintient cette composition au contact des fibres pendant un temps suffisant pour développer une coloration à l'aide d'un système oxydant et qu'on rince ensuite.

**9.** Procédé selon la revendication 8, caractérisé par le fait que l'on applique sur les fibres une composition (A) contenant dans un milieu approprié pour la teinture, au moins une indoline telle que définie dans la revendication 1

ou 2, la couleur étant révélée à l'aide d'un système oxydant chimique constitué par:

(i) des ions iodure et du peroxyde d'hydrogène, la composition (A) contenant dans ce cas, en plus, soit des ions iodure, soit du peroxyde d'hydrogène et l'application de la composition (A) est précédée ou suivie par l'application d'une composition (B) qui contient dans un milieu approprié pour la teinture, soit :

(a) du peroxyde d'hydrogène à un pH compris entre 2 et 12 et de préférence entre 2 et 7, lorsque la composition (A) contient des ions iodure, soit :
(b) des ions iodure à un pH compris entre 3 et 11 lorsque la composition (A) contient du peroxyde d'hydrogène;

(ii) des nitrites, l'application de la composition (A) étant suivie par l'application d'une composition aqueuse (B) présentant un pH acide, la composition (A) ou la composition (B) contenant au moins un nitrite;
(iii) des oxydants choisis parmi le peroxyde d'hydrogène, l'acide périodique et ses sels hydrosolubles, le métapériodate de sodium, l'hypochlorite de sodium, la chloramine T, la chloramine B, le ferricyanure de potassium, l'oxyde d'argent, le réactif de Fenton, l'oxyde de plomb (IV), le sulfate de cesium, le persulfate d'ammonium, les chlorites alcalins; ces oxydants étant présents dans la composition (A) contenant l'indoline de formule (I) ou appliqués simultanément ou séquentiellement au moyen d'une composition (B) les contenant dans un milieu approprié pour la teinture;
(iv) des anions d'un métal choisis parmi les permanganates ou bichromates, ces oxydants étant appliqués au moyen d'une composition (B) aqueuse, à un pH de 2 à 10, avant l'application de la composition (A);
(v) des sels métalliques des groupes 3 à 8 du tableau périodique, ces sels métalliques étant appliqués dans une étape séparée au moyen d'une composition (B) contenant ces sels dans un milieu approprié pour la teinture;
(vi) des sels de terres rares, ces sels de terres rares étant appliqués au moyen d'une composition (B) contenant ces sels dans un milieu approprié pour la teinture, la composition (B) étant appliquée préalablement ou postérieurement à l'application de la composition (A);
(vii) un dérivé quinonique choisi parmi les ortho- ou parabenzoquinones, les ortho- ou parabenzoquinones monoïmines ou diimines, les 1,2- ou 1,4-naphtoquinones, les ortho- ou parabenzoquinones sulfonimides, les $\alpha$, $\omega$-alkylène-bis-1,4-benzoquinones ou les 1,2 ou 1,4-naphtoquinones monoïmines ou diimines, l'indoline de formule (I) et les dérivés quinoniques étant choisis de façon à ce que la différence de potentiel d'oxydoréduction $\Delta E$ entre le potentiel d'oxydoréduction Ei de l'indoline de formule (I) déterminé à pH 7 en milieu phosphate sur électrode au carbone vitreux par voltamétrie et le potentiel d'oxydoréduction Eq du dérivé quinonique déterminé à pH 7 en milieu phosphate par polarographie sur électrode de mercure par rapport à l'électrode au calomel saturé, soit telle que :

$$\Delta E = Ei - Eq \leq 320 \text{ millivolts};$$

la composition (B) étant appliquée préalablement ou postérieurement à l'application de la composition (A).

**10.** Procédé selon la revendication 9, caractérisé par le fait que les ions iodure sont présents dans la composition (A) ou (B) dans des proportions comprises entre 0,007 et 4% en poids exprimées en ions $I^-$ par rapport au poids total de la composition (A) ou (B).

**11.** Procédé selon la revendication 9, caractérisé par le fait que les agents oxydants (iii) sont présents dans des proportions comprises entre 0,004 et 0,7 mole et de préférence entre 0,01 et 0,04 mole pour 100 g de composition.

**12.** Procédé selon la revendication 9, caractérisé par le fait que l'on applique sur les fibres kératiniques une composition (A) contenant dans un milieu approprié pour la teinture, une indoline de formule (I) et que l'on applique avant ou après la composition (A) une composition (B) contenant dans un milieu approprié pour la teinture, un sel métallique choisi parmi les sels de manganèse, de cobalt, de fer, de cuivre et d'argent utilisés dans des proportions comprises entre 0,01 et 2% en poids exprimé en ions métalliques par rapport au poids total de la composition.

**13.** Procédé selon la revendication 9, caractérisé par le fait que lorsqu'on utilise comme moyen oxydant une composition à base de peroxyde d'hydrogène, la teneur en peroxyde d'hydrogène dans la composition est comprise entre 1 et 40 volumes, de préférence entre 2 et 10 volumes.

**14.** Agent de teinture des fibres kératiniques, en particulier des fibres kératiniques humaines, à plusieurs composants, caractérisé par le fait qu'il comprend un premier composant constitué par une composition (A) contenant une indoline de formule (I) telle que définie dans la revendication 9, et un second composant constitué par l'une des compositions (B) définie dans l'une quelconque des revendications 9 à 13.

**Patentansprüche**

**1.** Indolinverbindung der Formel:

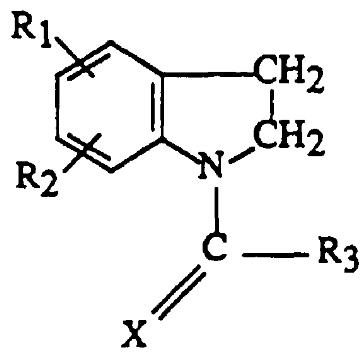

(I),

worin bedeuten:

die Gruppen $R_1$ und $R_2$ unabhängig voneinander Wasserstoff, $C_{1-4}$-Alkyl, $C_{1-4}$-Monohydroxyalkyl, $C_{2-4}$-Polyhydroxyalkyl, Nitro, Hydroxy, $C_{1-4}$ Alkoxy oder NHR, wobei R Wasserstoff, $C_{1-4}$-Alkyl, $C_{1-4}$-Monohydroxyalkyl oder $C_{2-4}$-Polyhydroxyalkyl bedeuten kann;
X ein Sauerstoffatom oder ein Schwefelatom;
$R_3$ die Gruppen $-OR_4$ oder $NR_5R_6$, worin:

$R_4$ eine geradkettige oder verzweigte $C_{1-10}$-Alkylgruppe, eine geradkettige oder verzweigte $C_{2-10}$-Alkenylgruppe, Benzyl, Alkoxyalkyl (wobei die Alkyl- und Alkoxygruppe 1 bis 4 Kohlenstoffatome aufweisen) oder $C_{1-4}$-Halogenalkyl bedeutet; und
$R_5$ und $R_6$ unabhängig voneinander eine $C_{1-4}$-Alkylgruppe bedeuten; mit der Maßgabe, daß $R_1$ und $R_2$ nicht gleichzeitig ein Wasserstoffatom oder eine Nitrogruppe bedeuten können und daß, wenn $R_1$ Alkyl, Alkoxy, Mono-oder Polyhydroxyalkyl bedeutet, $R_2$ eine Hydroxygruppe oder NHR ist, wobei R die oben angegebenen Bedeutungen aufweist;

wobei die Verbindungen 6-Nitro-1-N,N-dimethylcarbamoylindolin, 5-Nitro-1-N,N-dimethylcarbamoylindolin und 5-Amino-1-N,N-dimethylcarbamoylindolin ausgenommen sind.

**2.** Verbindungen nach Anspruch 1, dadurch gekennzeichnet, daß sie ausgewählt sind unter:

5,6-Dihydroxy-1-benzyloxycarbonylindolin,
5,6-Dihydroxy-1-ethyloxycarbonylindolin,
5,6-Dihydroxy-1-isopropyloxycarbonylindolin,
5,6-Dihydroxy-1-allyloxycarbonylindolin,
5,6-Dihydroxy-1-(2,2,2-trichlor)-ethoxycarbonylindolin,
5,6-Dihydroxy- 1-(2-ethyl)-hexyloxycarbonylindolin,
5,6-Dihydroxy-1-methoxyethoxycarbonylindolin,
5,6-Dihydroxy-1-dimethylcarbamoylindolin,
4-Hydroxy-5-methoxy-1-benzyloxycarbonylindolin,
6-Hydroxy-7-methoxy-1-benzyloxycarbonylindolin,
5,6-Dihydroxy-1-ethylthiocarbonylindolin,
5-Methoxy-6-hydroxy-1-benzyloxycarbonylindolin.

**3.** Färbemittelzusammensetzung, die dazu vorgesehen ist, zum Färben von Keratinfasem und insbesondere menschlichen Keratinfasern verwendet zu werden, dadurch gekennzeichnet, daß sie in einem zum Färben geeigneten Medium mindestens ein Indolin der folgenden Formel enthält:

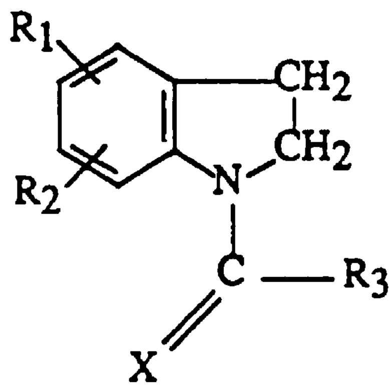

(I),

worin bedeuten:

die Gruppen $R_1$ und $R_2$ unabhängig voneinander Wasserstoff, $C_{1-4}$-Alkyl, $C_{1-4}$-Monohydroxyalkyl, $C_{2-4}$-Polyhydroxyalkyl, Nitro, Hydroxy, $C_{1-4}$ Alkoxy oder NHR, wobei R Wasserstoff, $C_{1-4}$-Alkyl, $C_{1-4}$-Monohydroxyalkyl oder $C_{2-4}$-Polyhydroxyalkyl bedeuten kann;
X ein Sauerstoffatom oder ein Schwefelatom;
$R_3$ die Gruppen $-OR_4$ oder $NR_5R_6$, worin:

$R_4$ eine geradkettige oder verzweigte $C_{1-10}$-Alkylgruppe, eine geradkettige oder verzweigte $C_{2-10}$-Alkenylgruppe, Benzyl, Alkoxyalkyl (wobei die Alkyl- und Alkoxygruppe 1 bis 4 Kohlenstoffatome aufweisen) oder $C_{1-4}$-Halogenalkyl bedeutet; und
$R_5$ und $R_6$ unabhängig voneinander eine $C_{1-4}$-Alkylgruppe bedeuten; mit der Maßgabe, daß $R_1$ und $R_2$ nicht gleichzeitig ein Wasserstoffatom oder eine Nitrogruppe bedeuten können und daß, wenn $R_1$ Alkyl, Alkoxy, Mono-oder Polyhydroxyalkyl bedeutet, $R_2$ eine Hydroxygruppe oder NHR ist, wobei R die oben angegebenen Bedeutungen aufweist;

**4.** Zusammensetzung nach Anspruch 3, dadurch gekennzeichnet, daß das Indolin in der Zusammensetzung in Mengenanteilen von 0,01 bis 8 Gew.-%, bezogen auf das Gesamtgewicht der Zusammensetzung, und vorzugsweise 0,03 bis 5 Gew.-% vorliegt.

**5.** Zusammensetzung nach Anspruch 3 oder 4, dadurch gekennzeichnet, daß das zum Färben geeignete Medium ein wäßriges Medium ist, das aus Wasser oder einem Gemisch von Wasser und einem oder mehreren Lösemitteln besteht.

**6.** Zusammensetzung nach einem der Ansprüche 3 bis 5, dadurch gekennzeichnet, daß die Zusammensetzung ferner mindestens einen Hilfsstoff enthält, der unter den Fettamiden, anionischen, kationischen, nichtionischen oder amphoteren grenzflächenaktiven Stoffen unter deren Gemischen, Verdickungsmitteln, Parfüms, Maskierungsmitteln, Filmbildnern, Behandlungsmitteln, Dispergiermitteln, Konditioniermitteln, Konservierungsmitteln, Trübungsmitteln, Quellmitteln für Keratinfasern oder deren Gemischen ausgewählt ist.

**7.** Zusammensetzung nach einem der Ansprüche 3 bis 6, dadurch gekennzeichnet, daß der pH-Wert der Zusammensetzung im Bereich von 4 bis 12 liegt.

**8.** Verfahren zum Färben von Keratinfasem und insbesondere menschlichen Keratinfasem, dadurch gekennzeichnet, daß auf die Fasern mindestens eine Zusammensetzung nach einem der Ansprüche 3 bis 7 aufgebracht wird, die Zusammensetzung während einer Zeitspanne mit den Fasern in Kontakt belassen wird, die ausreichend ist, um mit einem oxidierenden System eine Färbung zu entwickeln, und anschließend gespült wird.

**9.** Verfahren nach Anspruch 8, dadurch gekennzeichnet, daß auf die Fasern eine Zusammensetzung (A) aufgebracht

wird, die in einem zum Färben geeigneten Medium mindestens ein Indolin nach Anspruch 1 oder 2 enthält, wobei die Farbe mit einem chemischen oxidierenden System entwickelt wird, das besteht aus:

(i) Iodidionen und Wasserstoffperoxid, wobei die Zusammensetzung (A) in diesem Fall ferner entweder Iodidionen oder Wasserstoffperoxid enthält und vor oder nach dem Auftragen der Zusammensetzung (A) eine Zusammensetzung (B) aufgetragen wird, die in einem zum Färben geeigneten Medium enthält: entweder

(a) Wasserstoffperoxid bei einem pH-Wert im Bereich von 2 bis 12 und vorzugsweise 2 bis 7, wenn die Zusammensetzung (A) Iodidionen enthält, oder
(b) Iodidionen bei einem pH-Wert im Bereich von 3 bis 11, wenn die Zusammensetzung (A) Wasserstoffperoxid enthält.;

(ii) Nitriten, wobei nach dem Auftragen der Zusammensetzung (A) eine wäßrige Zusammensetzung (B) aufgetragen wird, die einen sauren pH-Wert aufweist, wobei die Zusammensetzung (A) oder die Zusammensetzung (B) mindestens ein Nitrit enthält;

(iii) Oxidationsmitteln, die unter Wasserstoffperoxid, Periodsäure und ihren wasserlöslichen Salzen, Natriummetaperiodat, Natriumhypochlorit, Chloramin T, Chloramin B, Kaliumferricyanid, Silberoxid, Fentons Reagenz, Blei(IV)oxid, Cäsiumsulfat, Ammoniumpersulfat und Alkalichloriten ausgewählt sind, wobei die Oxidationsmittel in der Zusammensetzung (A), die das Indolin der Formel (I) enthält, vorliegen oder gleichzeitig oder getrennt davon anschließend mit einer Zusammensetzung (B) aufgetragen werden, die sie in einem zum Färben geeigneten Medium enthält;

(iv) Anionen eines Metalls, die unter den Permanganaten oder Dichromaten ausgewählt sind, wobei diese Oxidationsmittel mit einer wäßrigen Zusammensetzung (B) bei einem pH-Wert von 2 bis 10 aufgebracht werden, bevor die Zusammensetzung (A) aufgetragen wird;

(v) Salzen von Metallen der Gruppen 3 bis 8 des Periodensystems, wobei die Metallsalze in einem getrennten Schritt mit einer Zusammensetzung (B) aufgebracht werden, die sie in einem zum Färben geeigneten Medium enthält;

(vi) Salze von Seltenerdmetallen, wobei die Seltenerdmetallsalze mit einer Zusammensetzung (B) aufgebracht werden, die sie in einem zum Färben geeigneten Medium enthält, wobei die Zusammensetzung (B) vor oder nach dem Auftragen der Zusammensetzung (A) aufgebracht wird;

(vii) einem Chinonderivat, das unter den o- oder p-Benzochinonen, o- oder p-Benzochinonmonoiminen oder -diiminen, 1,2- oder 1,4-Naphthochinonen, o- oder p-Benzochinonsulfonimiden, $\alpha,\omega$-Alkylen-bis-1,4-benzochinonen oder 1,2- oder 1,4-Naphthochinonmonoiminen oder -diiminen ausgewählt ist, wobei das Indolinderivat und die Chinonderivate so ausgewählt sind, daß der Unterschied im Redoxpotential AE des Redoxpotentials Ei des Indolins der Formel (I), das bei pH 7 in einem Phosphatmedium an einer Glas-Kohlenstoff-Elektrode voltametrisch bestimmt wurde, und des Redoxpotentials Eq des Chinonderivats, das bei pH 7 in einem Phosphatmedium an einer Quecksilberelektrode polarographisch in bezug auf eine gesättigte Kalomel-Elektrode bestimmt wurde, die folgende Beziehung erfüllt:

$$\Delta E = Ei - Eq \leq 320 \text{ mV},$$

wobei die Zusammensetzung (B) vor oder nach der Zusammensetzung (A) aufgetragen wird.

**10.** Verfahren nach Anspruch 9, dadurch gekennzeichnet, daß die Iodidionen in der Zusammensetzung (A) oder (B) in Mengenanteilen von 0,007 bis 4 Gew.-%, ausgedrückt als $I^-$-Ionen, bezogen auf das Gesamtgewicht der Zusammensetzung (A) oder (B), vorliegen.

**11.** Verfahren nach Anspruch 9, dadurch gekennzeichnet, daß die Oxidationsmittel (iii) in Mengenanteilen im Bereich von 0,004 bis 0,7 mol und vorzugsweise 0,01 bis 0,04 mol pro 100 g Zusammensetzung vorliegen.

**12.** Verfahren nach Anspruch 9, dadurch gekennzeichnet, daß auf die Keratinfasern eine Zusammensetzung (A), die in einem zum Färben geeigneten Medium ein Indolin der Formel (I) enthält, und vor oder nach der Zusammensetzung (A) eine Zusammensetzung (B) aufgetragen wird, die in einem zum Färben geeigneten Medium ein Metallsalz enthält, das unter den Salzen von Mangan, Cobalt, Eisen, Kupfer und Silber ausgewählt ist und das in Mengenanteilen im Bereich von 0,01 bis 2 Gew.-%, ausgedrückt als Metallionen, bezogen auf das Gesamtgewicht der Zusammensetzung, verwendet wird.

**13.** Verfahren nach Anspruch 9, dadurch gekennzeichnet, daß der Gehalt an Wasserstoffperoxid in der Zusammensetzung im Bereich von 1 bis 40 Volumina und vorzugsweise 2 bis 10 Volumina liegt, wenn als Oxidationsmittel eine Zusammensetzung auf der Basis von Wasserstoffperoxid verwendet wird.

**14.** Mittel zum Färben von Keratinfasern und insbesondere menschlichen Keratinfasern mit mehreren Komponenten, dadurch gekennzeichnet, daß es eine erste Komponente, die aus einer Zusammensetzung (A) mit dem Indolinderivat der Formel (1) nach Anspruch 9 besteht, und eine zweite Komponente enthält, die aus einer der in einem der Ansprüche 9 bis 13 definierten Zusammensetzungen (B) besteht.

## Claims

**1.** Indoline compound of formula:

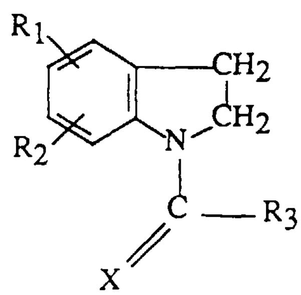

(I)

in which:

$R_1$ and $R_2$, independently of one another, represent a hydrogen atom or a $C_1$-$C_4$ alkyl, $C_1$-$C_4$ monohydroxyalkyl, $C_2$-$C_4$ polyhydroxyalkyl, nitro, hydroxyl, $C_1$-$C_4$ alkoxy or NHR group, it being possible for R to represent a hydrogen atom or a $C_1$-$C_4$ alkyl, $C_1$-$C_4$ monohydroxyalkyl or $C_2$-$C_4$ polyhydroxyalkyl group;
X represents an oxygen atom or a sulphur atom;
$R_3$ represents the -$OR_4$ or $NR_5R_6$ groups, in which:
$R_4$ represents a linear or branched $C_1$-$C_{10}$ alkyl, linear or branched $C_2$-$C_{10}$ alkenyl, benzyl, alkoxyalkyl (the alkyl and alkoxy groups having from 1 to 4 carbon atoms) or $C_1$-$C_4$ haloalkyl radical;
$R_5$ and $R_6$, independently of one another, represent a $C_1$-$C_4$ alkyl;

with the proviso that $R_1$ and $R_2$ cannot simultaneously denote a hydrogen atom or a nitro group and that when $R_1$ denotes an alkyl, alkoxy or mono- or polyhydroxyalkyl radical, $R_2$ represents a hydroxyl or NHR radical, R having the meanings shown above; with the exception of the compounds 6-nitro-1-N,N-dimethylcarbamoylindoline; 5-nitro-1-N,N-dimethylcarbamoylindoline and 5-amino-1-N,N-dimethylcarbamoylindoline.

**2.** Compounds according to Claim 1, characterized in that they are chosen from:

- 5,6-dihydroxy-1-benzyloxycarbonylindoline,
- 5,6-dihydroxy-l-ethyloxycarbonylindoline,
- 5,6-dihydroxy-1-isopropyloxycarbonylindoline,
- 5,6-dihydroxy-1-allyloxycarbonylindoline,
- 5,6-dihydroxy-1-(2,2,2-trichloro)ethoxycarbonylindoline [sic],
- 5,6-dihydroxy-1-(2-ethyl)hexyloxycarbonylindoline [sic],
- 5,6-dihydroxy-1-methoxyethoxycarbonylindoline,
- 5,6-dihydroxy-1-dimethylcarbamoylindoline,
- 4-hydroxy-5-methoxy-1-benzyloxycarbonylindoline,

- 6-hydroxy-7-methoxy-1-benzyloxycarbonylindoline,
- 5,6-dihydroxy-1-ethylthiocarbonylindoline,
- 5-methoxy-6-hydroxy-1-benzyloxycarbonylindoline.

**3.** Dyeing composition intended to be used for dyeing keratinous fibres, in particular human keratinous fibres, characterized in that it contains, in a medium suitable for dyeing, at least one indoline of formula:

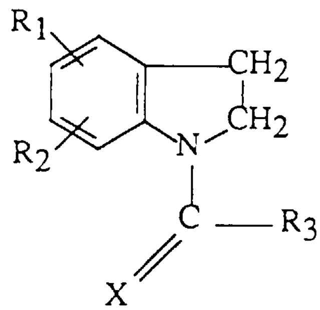

(I)

in which:

$R_1$ and $R_2$, independently of one another, represent a hydrogen atom or a $C_1$-$C_4$ alkyl, $C_1$-$C_4$ monohydroxyalkyl, $C_2$-$C_4$ polyhydroxyalkyl, nitro, hydroxyl, $C_1$-$C_4$ alkoxy or NHR group, it being possible for R to represent a hydrogen atom or a $C_1$-$C_4$ alkyl, $C_1$-$C_4$ monohydroxyalkyl or $C_2$-$C_4$ polyhydroxyalkyl group;
X represents an oxygen atom or a sulphur atom;
$R_3$ represents the $-OR_4$ or $NR_5R_6$ groups, in which:
$R_4$ represents a linear or branched $C_1$-$C_{10}$ alkyl, linear or branched $C_2$-$C_{10}$ alkenyl, benzyl, alkoxyalkyl (the alkyl and alkoxy groups having from 1 to 4 carbon atoms) or $C_1$-$C_4$ haloalkyl radical;
$R_5$ and $R_6$, independently of one another, represent a $C_1$-$C_4$ alkyl;

with the proviso that $R_1$ and $R_2$ cannot simultaneously denote a hydrogen atom or a nitro group and that when $R_1$ denotes an alkyl, alkoxy or mono- or polyhydroxyalkyl radical, $R_2$ represents a hydroxyl or NHR radical, R having the meanings shown above.

**4.** Composition according to Claim 3, characterized in that the indoline is present in the composition in proportions between 0.01 and 8% by weight with respect to the total weight of the composition and preferably between 0.03 and 5% by weight.

**5.** Composition according to Claim 3 or 4, characterized in that the medium suitable for dyeing is an aqueous medium consisting of water or a water/solvent(s) mixture.

**6.** Composition according to any one of Claims 3 to 5, characterized in that the composition additionally contains at least one adjuvant chosen from fatty amides, anionic, cationic, nonionic or amphoteric surface-active agents or their mixtures, thickening agents, fragrances, sequestering agents, film-forming agents, treatment agents, dispersing agents, conditioning agents, preserving agents, opacifying agents, agents for swelling keratinous fibres or their mixtures.

**7.** Composition according to any one of Claims 3 to 6, characterized in that the pH of the composition is between 4 and 12.

**8.** Process for dyeing keratinous fibres, in particular human keratinous fibres, characterized in that there is applied to these fibres at least one composition as defined in any one of Claims 3 to 7, in that this composition is maintained in contact with the fibres for a time sufficient to develop a colouring with the aid of an oxidizing system and in that rinsing is subsequently carried out.

**9.** Process according to Claim 8, characterized in that there is applied to the fibres a composition (A) containing, in a medium suitable for dyeing, at least one indoline as defined in Claim 1 or 2, the colour being developed with the aid of a chemical oxidizing system consisting of:

(i) iodide ions and hydrogen peroxide, the composition (A) additionally containing in this case either iodide ions or hydrogen peroxide and the application of the composition (A) is preceded or followed by the application of a composition (B) which contains, in a medium suitable for dyeing, either:

(a) hydrogen peroxide at a pH between 2 and 12 and preferably between 2 and 7, when the composition (A) contains iodide ions, or
(b) iodide ions at a pH between 3 and 11, when the composition (A) contains hydrogen peroxide;

(ii) nitrites, the application of the composition (A) being followed by the application of an aqueous composition (B) having an acidic pH, the composition (A) or the composition (B) containing at least one nitrite;
(iii) oxidizing agents chosen from hydrogen peroxide, periodic acid and its water-soluble salts, sodium metaperiodate, sodium hypochlorite, chloramine-T, chloramine-B, potassium ferricyanide, silver oxide, Fenton's reagent, lead(IV) oxide, caesium sulfate, ammonium persulphate, or alkali metal chlorites; these oxidizing agents being present in the composition (A) containing the indoline of formula (I) or applied simultaneously or sequentially by means of a composition (B) containing them in a medium suitable for dyeing;
(iv) anions of a metal chosen from permanganates or dichromates, these oxidizing agents being applied by means of an aqueous composition (B), at a pH of 2 to 10, before the application of the composition (A);
(v) metal salts of groups 3 to 8 of the periodic table, these metal salts being applied in a separate stage by means of a composition (B) containing these salts in a medium suitable for dyeing;
(vi) rare-earth metal salts, these rare-earth metal salts being applied by means of a composition (B) containing these salts in a medium suitable for dyeing, the composition (B) being applied prior to or subsequent to the application of the composition (A);
(vii) a quinone derivative chosen from ortho-or parabenzoquinones, monoimines or diimines of ortho-or parabenzoquinones, 1,2- or 1,4-naphthoquinones, sulphonimides of ortho- or parabenzoquinones, $\alpha,\omega$-alkylenebis-1,4-benzoquinones, or monoimines or diimines of 1,2- or 1,4-naphthoquinones, the indoline of formula (I) and the quinone derivatives being chosen so that the difference in oxidation-reduction potential $\Delta E$ between the oxidation-reduction potential Ei of the indoline of formula (I), determined at pH 7 in phosphate medium on a vitreous carbon electrode by voltametry, and the oxidation-reduction potential Eq of the quinone derivative, determined at pH 7 in phosphate medium by polarography on a mercury electrode with reference to the saturated calomel electrode, is such that:

$$\Delta E = Ei\text{-}Eq \leq 320 \text{ millivolts;}$$

the composition (B) being applied prior to or subsequent to the application of the composition (A).

**10.** Process according to Claim 9, characterized in that the iodide ions are present in the composition (A) or (B) in proportions between 0.007 and 4% by weight, expressed as $I^-$ ions, with respect to the total weight of the composition (A) or (B).

**11.** Process according to Claim 9, characterized in that the oxidizing agents (iii) are present in proportions between 0.004 and 0.7 mol, and preferably between 0.01 and 0.04 mol, per 100 g of composition.

**12.** Process according to Claim 9, characterized in that there is applied to keratinous fibres a composition (A) containing, in a medium suitable for dyeing, an indoline of formula (I) and in that there is applied, before or after the composition (A), a composition (B) containing, in a medium suitable for dyeing, a metal salt chosen from manganese, cobalt, iron, copper and silver salts used in proportions between 0.01 and 2% by weight, expressed as metal ions, with respect to the total weight of the composition.

**13.** Process according to Claim 9, characterized in that, when a composition based on hydrogen peroxide is used as oxidizing means, the hydrogen peroxide content in the composition is between 1 and 40 volumes, preferably between 2 and 10 volumes.

**14.** Multi-component agent for dyeing keratinous fibres, in particular human keratinous fibres, characterized in that it comprises a first component consisting of a composition (A) containing an indoline of formula (I) as defined in Claim 9 and a second component consisting of one of the compositions (B) defined in any one of Claims 9 to 13.